# FASCICULE DE BREVET EUROPEEN

(11) **EP 1 334 725 B1**
(45) Date de publication et mention de la délivrance du brevet: **14.02.2007**
(21) Numéro de dépôt: 03008979.1
(22) Date de dépôt: 08.10.1997
(51) Int. Cl.: A61K 31/57, A61K 31/565, A61P 15/18

(54) **Utilisation d'une composition estroprogestative en tant que contraceptif oral**
Östroprogestatives Antikonzeptionsmittel
Estroprogestative contraceptive composition

(30) Priorité: 08.10.1996 FR 9612239
(43) Date de publication de la demande: 13.08.2003
(62) Demande divisionnaire de: 97944940.2
(73) Titulaire: LABORATOIRE THERAMEX, 98000 Monaco (MC)
(72) Inventeur: Lanquetin, Michel, Laghet, 06340 La Trinité (FR); Paris, Jacques, Le Clos de Cimiez, 06100 Nice (FR); Thomas, Jean-Louis, 94220 Charenton le Pont (FR)
(74) Mandataire: Gillard, Marie-Louise

(56) Documents cités:
- CONARD J ET AL: "Cardiovascular risk factors and combined estrogen-progestin replacement therapy: A placebo-controlled study with nomegestrol acetate and estradiol" FERTILITY AND STERILITY, 64 (5). 1995. 957-962., XP000675866
- SITRUK-WARE R: "[Pharmacology of oral contraceptives]" REV PRAT, DEC 1 1995, 45 (19) P2401-6, XP000675852 FRANCE

## Description

La présente invention se rapporte au domaine de la chimie thérapeutique et plus particulièrement au domaine de la technique pharmaceutique hormonale.

Elle a plus précisément pour objet l'utilisation de compositions pharmaceutiques formées d'une association estroprogestative afin de bloquer l'ovulation chez la femme en période d'activité ovarienne.

Cette association est destinée à être administrée par voie orale.

Le progestatif utilisé est l'acétate de nomégestrol qui est actif par voie orale.

L'estrogène utilisé est l'estradiol libre ou estérifié, ou des estrogènes conjugués équins se présentant selon une formulation active par voie orale, et notamment le valérate d'estradiol.

L'acétate de nomégestrol et l'estradiol libre ou estérifié, ou les estrogènes conjugués équins sont administrés sous une des formes permettant l'administration par voie orale : gélules, capsules, pilules, sachets de poudre, comprimés, comprimés enrobés, dragées, etc...

La présente invention a pour objet l'utilisation d'une association estroprogestative, active par voie orale, administrée d'une manière combinée dans le blocage de l'ovulation chez la femme en période d'activité ovarienne.

L'association estroprogestative comprenant de l'acétate de nomégestrol et un estrogène a été décrite par CONARD et al. dans Fertility and Sterility vol.64, n°5, novembre 1995, p.957-961 dans le cadre d'un traitement hormonal substitutif de la ménopause.

Les compositions à base d'acétate de nomégestrol et d'estradiol libre ou estérifié, ou d'estrogènes conjugués équins sont administrés de façon continue ou intermittente, de 21 à 25 jours par mois.

Selon un mode d'exécution particulier, les compositions contiennent une quantité d'acétate de nomégetrol s'échelonnant de 1,5 à 3,75 mg et une quantité d'estradiol libre ou estérifié, ou d'estrogènes conjugués équins s'échelonnant de 0,5 à 3 mg. De préférence, les formulations optimales contiennent 2,5 mg d'acétate de nomégestrol associé à : soit 1,5 mg d'estradiol libre ou 2 mg d'ester d'estradiol ou 0,625 mg d'estrogènes conjugués équins, par prise journalière.

Chez les femmes en période d'activité ovarienne, jeunes ou dans les années précédant la ménopause, l'administration cyclique de la combinaison hormonale est capable d'inhiber l'ovulation et d'exercer un effet contraceptif dans la mesure où il a été prouvé que l'acétate de nomégestrol était capable d'inhiber le pic ovulatoire de LH et de FSH, à partir de 1,25 mg/jour (BAZIN B. et al., Effect of nomegestrol acetate, a new 19-norprogesterone derivative on pituitary ovarian function in women. Br. J. Obstet. Gynaecol, 1987, 94 : 1199-1204). Lorsque la combinaison hormonale est donnée dans un but contraceptif, l'acétate de nomegestrol a pour but de bloquer l'ovulation et la composante estrogénique de compenser l'hypoestrogénie et d'assurer un meilleur contrôle du cycle.

Le procédé d'obtention des compositions selon l'invention consiste à mélanger les principes actifs : acétate de nomégestrol et estradiol libre ou estérifié, ou des estrogènes conjugués équins avec un ou des excipients inertes, non toxiques, pharmaceutiquement acceptables.

Parmi les excipients on pourra citer les agents liants et solubilisants, les agents de compression, les agents de désintégration et les agents de glissement.
On peut soumettre ce mélange à une compression directe ou en plusieurs étapes pour former des comprimés que l'on peut protéger en surface, si désiré par pelliculage, laquage ou enrobage. La production de comprimés par compression directe permet de réduire au maximum la proportion d'agents de dilution, d'agents liants, d'agents de désintégration et d'agents de glissement.

La production de gélules pourra se faire en mélangeant les principes actifs à un diluant inerte et un agent de glissement.

Les comprimés renferment, en particulier, des agents de dilution de la masse comme le lactose, le sorbitol pour compression directe, commercialisé sous la dénomination NEOSORB 60, le Palatinite qui est la marque déposée pour désigner un mélange équimolaire d'isomère de -D-glucopyranosido 1,6-mannitol et de -D-glucopyranosido 1,6-glucitol cristallisé avec deux molécules d'eau, le mannitol, le sorbitol ou le mélange lactose/PVP vendu sous la dénomination Ludipress.
Les agents liants de compression sont en général des celluloses microcristallines comme celles vendues sous la dénomination AVICEL PH 101 ou AVICEL PH 102.
La polyvinylpyrrolidone joue également un rôle important et facilite l'agglomération des poudres et la compressibilité de la masse. On utilise à cette fin des polyvinylpyrrolidones de poids moléculaire compris entre 10000 et 30000 comme la Povidone, le Kollidon de grade compris entre 12 et 30.

Le mélange contient également des agents de glissement ou anti-électrostatiques qui évitent que la poudre ne s'agglomère dans les trémies d'alimentation. On peut citer, à cet égard, les silices colloïdales vendues sous la dénomination AEROSIL 100 ou AEROSIL 200.

Le mélange contient aussi des agents de désintégration qui permettent une désintégration ou un délitement conforme aux normes pharmaceutiques. On pourra citer comme agents de désintégration utiles, les polymères de vinylpyrrolidones réticulées telles que celles vendues sous les dénominations Polyplasdone ou Polyclar AT, les carboxyméthylamidons comme ceux vendus sous les dénominations Amigel ou Explotab, les carboxyméthylcelluloses réticulées ou croscarmelloses comme le composé vendu sous la dénomination AC-DI-SOL.
En outre, la préparation contient des agents de lubrification qui facilitent la compression et l'éjection du comprimé sur les machines à comprimer. On pourra citer comme agents de lubrification, le palmitostéarate de glycérol vendu sous la dénomination Précirol, le stéarate de magnésium, l'acide stéarique ou le Talc.
Après compression, les comprimés peuvent être enrobés pour assurer leur conservation ou faciliter leur déglutition.
Les agents d'enrobage sont soit cellulosiques comme le phtalate de cellulose (Sepifilm, Pharmacoat), soit polyvinyliques du type Sépifilm ECL, soit saccharosiques comme le sucre pour dragéification du type Sépisperse DR, AS, AP ou K (colorés).

Les comprimés enrobés ou non, peuvent, en outre, être colorés en surface ou dans la masse, par des colorants végétaux ou synthétiques (par ex. laque au jaune de quinoléine ou E 104).
Les proportions des différents constituants varient selon la nature du comprimé à réaliser.
La teneur en principes actifs peut varier de 1,5 à 3,75 mg pour l'acétate de nomégestrol et de 0,5 à 3 mg pour l'estradiol libre ou estérifié ou pour les estrogènes conjugués équins. Les agents de dilution varient de 20 à 75 % de la masse totale, les agents de glissement de 0,1 à 2 % de la masse totale, les agents liants de compression varient de 2 à 20 %, la polyvinylpyrrolidone de 0,5 à 15 %, les agents de désintégration varient de 2 à 5,5 % pour la polyvinylpyrrolidone réticulée ou pour le carboxyméthylamidon, de 2,0 à 3,0 pour la croscarméllose.
Les quantités d'agents de lubrification varient en fonction de la nature de l'agent de 0,1 à 3,0 %.

Les compositions selon l'invention sont destinées à être administrées une fois par jour. Cependant, en fonction des besoins thérapeutiques, l'administration peut être fragmentée (deux fois par jour) ou bien au contraire, renouvelée (deux comprimés par jour).
L'exemple suivant illustre l'invention. Il ne la limite en aucune façon.

### EXEMPLE 1

**Comprimés à 4 mg de principes actifs**

| | | |
|---|---|---|
| Principes actifs : | - estradiol | 1,5 mg |
| | - acétate de nomégestrol | 2,5 mg |
| Cellulose microcristalline | | 22,4 mg |
| (*commercialisée sous la dénomination AVICEL PH 102*) | | |
| Lactose | | 60 mg |
| Polyvinylpyrrolidone | | 8,4 mg |
| Silice colloïdale | | 1,2 mg |
| Palmitostéarate de glycerol | | 3,6 mg |
| Colorant E.104 | | 0,4 mg |

| | | |
|---|---|---|
| pour un comprimé terminé au poids moyen de 100 mg. | | |

## Revendications

1. Utilisation d'une composition estroprogestative comprenant de l'acétate de nomégestrol et un estrogène en tant que contraceptif oral.

2. Utilisation selon la revendication 1, **caractérisée en ce que** l'estrogène est choisi parmi l'estradiol libre ou estérifié et les estrogènes équins.

3. Utilisation selon la revendication 1 ou 2, **caractérisée en ce que** l'estrogène est un ester d'estradiol, notamment le valérate d'estradiol.

4. Utilisation selon l'une des revendications 1 à 3, **caractérisée en ce que** la composition estroprogestative comprend 0,5 à 3 mg d'estrogène.

5. Utilisation selon l'une des revendications 1 à 4, **caractérisée en ce que** la composition estroprogestative comprend 1,5 à 3,75 mg d'acétate de nomégestrol.

6. Utilisation selon la revendication 5, **caractérisée en ce que** la composition estroprogestative comprend 2,5 mg d'acétate de nomégestrol.

7. Utilisation selon l'une des revendications 1 à 5, **caractérisée en ce que** la composition estroprogestative comprend 2,5 mg d'acétate de nomégestrol et 1,5 mg d'estradiol libre.

8. Utilisation selon l'une des revendications 1 à 5, **caractérisée en ce que** la composition estroprogestative comprend 2,5 mg d'acétate de nomégestrol et 2 mg d'ester d'estradiol.

9. Utilisation selon l'une des revendications 1 à 5, **caractérisée en ce que** la composition estroprogestative comprend 2,5 mg d'acétate de nomégestrol et 0,625 mg d'estrogènes conjugués équins.

## Claims

1. Use of an estroprogestative composition comprising nomegestrol acetate and an estrogen as oral contraceptive.

2. Use according to claim 1, **characterized in that** the estrogen is selected among free or esterified estradiol and conjugated equine estrogens.

3. Use according to claim 1 or 2, **characterized in that** the estrogen is an ester of estradiol, notably estradiol valerate.

4. Use according to any one of claims 1 to 3, **characterized in that** the estroprogestative composition comprises 0.5 to 3 mg of estrogen.

5. Use according to any one of claims 1 to 4, **characterized in that** the estroprogestative composition comprises 1.5 to 3.75 mg of nomegestrol acetate.

6. Use according to claim 5, **characterized in that** the estroprogestative composition comprises 2.5 mg of nomegestrol acetate.

7. Use according to any one of claims 1 to 5, **characterized in that** the estroprogestative composition comprises 2.5 mg of nomegestrol acetate and 1.5 mg of free estradiol.

8. Use according to any one of claims 1 to 5, **characterized in that** the estroprogestative composition comprises 2.5 mg of nomegestrol acetate and 2 mg of ester of estradiol.

9. Use according to any one of claims 1 to 5, **characterized in that** the estroprogestative composition comprises 2.5 mg of nomegestrol acetate and 0.625 mg of conjugated equine estrogens.

## Patentansprüche

1. Verwendung eines östroprogestativen Präparats, umfassend Nomegestrolacetat und ein Östrogen, als orales Antikonzeptionsmittel.

2. Verwendung gemäß Anspruch 1, **dadurch gekennzeichnet, dass** das Östrogen ausgewählt ist aus freiem oder verestertem Östradiol und konjugierten equinen Östrogenen.

3. Verwendung gemäß Anspruch 1 oder 2, **dadurch gekennzeichnet, dass** das Östrogen ein Östradiolester, inbesondere Östradiolvalerat, ist.

4. Verwendung gemäß einem der Ansprüche 1 bis 3, **dadurch gekennzeichnet, dass** das östroprogestative Präparat 0,5 bis 3 mg Östrogen umfasst.

5. Verwendung gemäß einem der Ansprüche 1 bis 4, **dadurch gekennzeichnet, dass** das östroprogestative Präparat 1,5 bis 3,75 mg Nomegestrolacetat umfasst.

6. Verwendung gemäß Anspruch 5, **dadurch gekennzeichnet, dass** das östroprogestative Präparat 2,5 mg Nomegestrolacetat umfasst.

7. Verwendung gemäß einem der Ansprüche 1 bis 5, **dadurch gekennzeichnet, dass** das östroprogestative Präparat 2,5 mg Nomegestrolacetat und 1,5 mg freies Östradiol umfasst.

8. Verwendung gemäß einem der Ansprüche 1 bis 5, **dadurch gekennzeichnet, dass** das östroprogestative Präparat 2,5 mg Nomegestrolacetat und 2 mg Östradiolester umfasst.

9. Verwendung gemäß einem der Ansprüche 1 bis 5, **dadurch gekennzeichnet, dass** das östroprogestative Präparat 2,5 mg Nomegestrolacetat und 0,625 mg konjugierte equine Östrogene umfasst.
